# EUROPÄISCHE PATENTSCHRIFT

(11) **EP 2 468 216 B1**
(45) Veröffentlichungstag und Bekanntmachung des Hinweises auf die Patenterteilung: **14.05.2014**
(21) Anmeldenummer: 10196739.6
(22) Anmeldetag: 23.12.2010
(51) Int. Cl.: A61F 2/36, A61F 2/38, A61F 2/28

(54) **Implantierbare Prothese zum Ersatz des menschlichen Hüft- oder Kniegelenks und der angrenzenden Knochenabschnitte**
Implantable prosthesis for replacing human hip or knee joints and the adjoining bone sections
Prothèse pouvant être implantée destinée à remplacer une articulation de hanche ou de genou humains et des sections d'os limitrophes

(43) Veröffentlichungstag der Anmeldung: 27.06.2012
(73) Patentinhaber: Baumgart, Rainer, Dipl.-Ing. Dr. med., 81479 München (DE)
(72) Erfinder: Baumgart, Rainer, Dipl.-Ing. Dr. med., 81479 München (DE)
(74) Vertreter: Hano, Christian

(56) Entgegenhaltungen:
- EP-A1- 1 371 346
- WO-A2-02/40092
- US-A1- 2004 254 646
- US-A1- 2010 241 239

## Beschreibung

Die Erfindung betrifft eine implantierbare Prothese zum Ersatz des menschlichen Hüft- oder Kniegelenks und der angrenzenden Knochenabschnitte, wobei die Prothese ein Gelenkersatzteil, ein Schaftersatzteil, welches mit dem Gelenkersatzteil verbindbar ist oder ein Stück damit bildet und einen zentralen Hohlraum hat, sowie ein in den Hohlraum einsetzbares stabförmiges Schaftverankerungsteil aufweist. Eine solche implantierbare Prothese ist aus der EP 1 371 346 B1 bekannt.

Nach Resektion eines Knochentumors kann der entfernte Knochen und das betroffene Gelenk durch eine Tumorprothese ersetzt werden und es besteht zunächst keine Beinlängendifferenz. Die Prothese wird in dem verbliebenen Knochen verankert, wie es beispielsweise in der EP 1 371 346 B1 beschrieben ist. Wenn sich wachstumsbedingt eine Beinlängendifferenz einstellt, kann der Knochen osteotomiert und die Prothese anstelle des Schaftverankerungsteils mit einem Distraktionsmarknagel zur Durchführung der Kallusdistraktionsmethode bestückt werden. Solche Distraktionsmarknägel sind beispielsweise in der EP 0 432 253 B1 beschrieben.

Wenn ein zweiter Verlängerungsschritt notwendig wird, muss diese Prozedur erneut durchgeführt werden. In jedem Fall muss aber nach Abschluss des Längenwachstums der Distraktionsmarknagel, der nicht zur dauerhaften Schaftverankerung belassen werden kann, durch ein solides Schaftverankerungsteil ersetzt werden. Der Wechsel von einem Distraktionsmarknagel oder der Austausch gegen ein solides Schaftverankerungsteil ist meist nur möglich, wenn die Gelenkkomponenten entkoppelt werden, so dass umfangreiche operative Freilegungen erforderlich sind. Somit sind mindestens zwei, meist jedoch sogar drei umfangreiche operative Eingriffe zusätzlich zu der Resektion des Tumors gelenknah im Bereich der Prothese erforderlich, was ein erhebliches Infektionsrisiko bedeutet.

In der US 2004/0254646 A1 ist eine implantierbare Prothese zum Ersatz des menschlichen Hüft- oder Kniegelenks und der angrenzenden Knochenabschnitte bekannt, die einen Gelenkersatzteil und Schaftersatzteil umfasst, das mit dem Gelenkersatzteil ein Stück damit bildet und einen zentralen Hohlraum hat. In den Hohlraum ist ein stabförmiger Schaftverankerungsteil einsetzbar. Der Schaftverankerungsteil weist an seinen beiden Seiten Konusabschnitte auf, auf die jeweils eine Kappe aufsetzbar ist, die mittels einer Befestigungsschraube fixiert werden kann, die in en Gewinde in dem Konusabschnitt eingreift. Die Kappe wird bei der Implantierung in einen Oberschenkelknochen eingesetzt.

Der Erfindung liegt deshalb die Aufgabe zugrunde, unter Beibehaltung der Vorteile der Knochenverlängerung nach der Kallusdistraktionsmethode bei liegender Tumorprothese das Ausmaß des erneuten operativen Eingriffs und damit das Infektionsrisiko deutlich zu vermindern, wobei das künstliche Gelenk selbst möglichst wenig tangiert werden soll.

Diese Aufgabe wird ausgehend von der implantierbaren Prothese der eingangs beschriebenen Art dadurch gelöst, dass das Schaftverankerungsteil auf der gelenkersatzteilabgewandten Seite eine Ortungseinrichtung zum Ausbilden eines Kanals durch den Knochenabschnitt auf der gelenkersatzteilabgewandten Seite von außen her axial fluchtend zu dem Schaftverankerungsteil und bis zu diesem hin und eine Befestigungseinrichtung für den Eingriff mit einem außerhalb des Knochenabschnitts befindlichen Werkzeug durch den Kanal hindurch zum Ausüben wenigstens einer Zugkraft auf das Schaftverankerungsteil aufweist, um es aus dem Knochenabschnitt durch den Kanal hindurch entfernen zu können.

Die am Schaftverankerungsteil vorgesehene Ortungseinrichtung ermöglicht somit das gezielte Bohren oder Fräsen eines Kanals in axial fluchtender Ausrichtung zu dem im Knochenabschnitt eingesetzten Schaftverankerungsteil und mit einem Durchmesser, der den Durchgang des Schaftverankerungsteils zulässt. Die Befestigungseinrichtung an der gelenkersatzteilabgewandten Stirnseite des Schaftverankerungsteils ermöglicht dann den Angriff eines Werkzeugs, mit dem das Schaftverankerungsteil aus dem Hohlraum des Schaftersatzteils heraus durch den gefrästen Kanal im Knochen gezogen werden kann. Anstelle des Schaftverankerungsteils kann dann durch den gefrästen Knochenkanal ein Distraktionsmarknagel in den Hohlraum des Schaftersatzteils eingeführt und in bekannter Weise fixiert werden. Nach Abschluss des gewünschten Längenwachstums des Knochens an einer vorher vorgenommenen Osteotomie kann der Distraktionsmarknagel wiederum durch den Kanal im Knochen entfernt und durch ein entsprechend langes massives Schaftverankerungsteil ausgetauscht werden, das dann Teil der Prothese bleibt. Diese Maßnahmen können alle über die gelenkersatzteilabgewandte Seite des Knochens ausgeführt werden, ohne dass erneute große Operationen im Bereich der Prothese erforderlich sind, so dass das Infektionsrisiko minimiert ist.

Die Ortungseinrichtung kann eine für den Patienten ungefährliche Strahlungsquelle sein, die dem Schaftverankerungsteil auf dessen gelenkersatzteilabgewandter Seite zugeordnet ist und durch deren Anmessung von außerhalb des Knochenabschnitts der Kanal in der benötigten Ausgestaltung ausgebildet werden kann.

Als Ortungseinrichtung kann vorteilhafterweise auch ein aus der Befestigungseinrichtung zentral vorstehendes drahtförmiges Führungselement verwendet werden, das durch den Knochenabschnitt hindurchgeht und aus diesem nach außen herausragt. Die Verbindung zwischen dem Führungselement und der Befestigungseinrichtung ist vorteilhafterweise lösbar ausgeführt. Dies kann beispielsweise dadurch erreicht werden, dass die Befestigungseinrichtung eine Innengewindebohrung und das Führungselement ein entsprechendes Außengewinde aufweist.

Um das Herausziehen des Schaftverankerungsteils durch den gefrästen Kanal unter der Zugwirkung des Werkzeugs zu erleichtern, ist das Schaftverankerungsteil zweckmäßigerweise so ausgebildet, dass durch das Werkzeug auch ein Drehmoment auf das Schaftverankerungsteil übertragbar ist.

Werkzeugseitig vor dem Innengewinde weist dafür das Schaftverankerungsteil zusätzlich einen Innenmehrkant auf. Das dabei verwendete Werkzeug ist dann zweigeteilt ausgebildet und hat diametral innen ein Element zur Zugkraftübertragung, beispielsweise eine Zugstange mit einem Außengewinde, und diametral außen ein Element zur Übertragung des Drehmoments auf das Schaftverankerungsteil, beispielsweise ein hülsenförmiges Verbindungselement mit einem Außenmehrkant.

Bei Verwendung eines Führungselements als Ortungseinrichtung wird dieses aus der die Befestigungseinrichtung bildenden Innengewindebohrung herausgeschraubt. Die Zugstange kann dann durch das hülsenförmige Verbindungselement hindurchgeführt und in die Innengewindebohrung der Befestigungseinrichtung am Schaftverankerungsteil eingeschraubt werden, um axialen Zug auf das Schaftverankerungsteil auszuüben, während sich über den Mehrkanteingriff von Verbindungselement und Befestigungseinrichtung ein Drehmoment von außen übertragen lässt, wodurch das Schaftverankerungsteil durch Zug und Drehmoment extrahiert werden kann.

An dem Schaftverankerungsteil ist vorteilhafterweise auf der Werkzeugbefestigungsseite eine Bohrschneide vorgesehen, die bei Drehung des Schaftverankerungsteils durch das außerhalb des Knochens befindliche Werkzeug eventuell in den gefrästen Kanal vorstehende Knochenteile abträgt und dadurch das Herausziehen des Schaftverankerungsteils erleichtert.

Die Oberfläche des Schaftverankerungsteils ist glatt, vorzugsweise poliert und hat zweckmäßigerweise eine Oberflächenrautiefe von 0,1 µm oder weniger. Dadurch wird ein Angriff des lebenden Knochens an dem Schaftverankerungsteil weitgehend ausgeschlossen, so dass das Herausziehen des Schaftverankerungsteils aus dem Markraum ohne großen Widerstand erfolgen kann.

Damit das Herausziehen des Schaftverankerungsteils aus dem Hohlraum des Schaftersatzteils problemlos vonstatten gehen kann, ist zweckmäßigerweise die Innenwand des Hohlraums gleitreibungsmindernd ausgebildet, beispielsweise durch Aufbringen einer dünnen Gleitschicht oder Einbringen einer Kunststoffhülse.

Vorzugsweise wird in dem Gelenkersatzteil oder dem Schaftersatzteil eine in den Hohlraum mündende Seitenbohrung vorgesehen, durch die ein Kabel durchgeführt werden kann, das mit seinem einen Ende an einer subkutanen Antenne zur Energieeinspeisung und an seinem anderen Ende mit einem Antrieb eines Distraktionsmarknagels verbindbar ist, wenn dieser nach Entfernen des Schaftverankerungsteils in das Schaftersatzteil eingeführt wird, um eine Knochendistraktion nach der Kallusdistraktionsmethode auszuführen.

An dem Schaftersatzteil können im Bereich seines gelenkersatzteilabgewandten Endes bereits bei der ersten Operation Verbindungseinrichtungen zur Fixierung an einem angrenzenden Knochenabschnitt angebracht werden, die jedoch erst bei der Ausführung einer Distraktion Bedeutung gewinnen.

Das Schaftverankerungsteil hat über seine Länge einen gleichbleibenden Querschnitt, kann sich aber auch zum Gelenkersatzteil hin verjüngen oder verjüngte Abschnitte aufweisen, wie dies auch bei einem Distraktionsmarknagel der Fall sein kann.

Das Schaftverankerungsteil kann ein massiver Stabilisator oder ein Marknagel mit integriertem Antrieb für die Distraktion sein.

In der erfindungsgemäßen Ausgestaltung kann die implantierbare Prothese mit ihrem gelenkbildenden Teil konventionell ausgeführt werden, was entscheidende Vorteile hat, weil das Prothesenschloss zur Tibiakomponente wesentlich einfacher und solider ausgeführt werden kann und die Prothese nicht mehr entkoppelt werden muss und dauerhaft belassen werden kann. Auch die Verbindung zu dem Schaftersatzteil, dessen Länge variabel und so kurz wie möglich ausgeführt werden kann, dass lediglich der durch die Resektion entstehende Defekt überbrückt wird, kann in konventioneller Form, beispielsweise als Konus- und Schraubverbindung zur Ausführung kommen.

Bei einem Ersatz des Kniegelenks und des kniegelenknahen Femurschaftteils wird nach einer En-Bloc-Resektion des erkrankten Knochenteils des Femurs zunächst die tibiale Gelenkkomponente implantiert. Hierzu kommt bei Kindern vorzugsweise eine Tibia-Plateaukomponente mit einem PTFE-Inlay zur Anwendung, deren Schaftverankerungsteil poliert ist, wobei die Rotationsstabilität über Zapfen unter der Tibiaplateaukomponente gegeben ist. Bei dieser Anordnung kann unter dem Druck der erhaltenen Wachstumsfuge das polierte Schaftverankerungsteil gleiten und das verbliebene Wachstumspotential der proximalen Tibiawachstumsfuge genutzt werden.

Anschließend wird in den Trochanter major in der Frontalebene in Verlängerung des Markraums und in der seitlichen Ebene auf Höhe des vorderen Drittelpunkts des proximalen Femurs ein Führungsdraht eingeführt und durch den Markraum von proximal bis in den Defekt vorgeschoben. Über diesen Führungsdraht wird der Markraum schrittweise von distal so weit aufgefräst, wie es für das Einführen des Schaftverankerungsteils erforderlich ist. Das Gelenkersatzteil und das Schaftersatzteil werden nun je nach Defektgröße ausgewählt. In den Hohlraum des Schaftersatzteils wird nun auf dessen offener Seite das gerade, auf den Markraumdurchmesser abgestimmte, außen polierte Schaftverankerungsteil eingeführt und formschlüssig mit Bolzen oder Schrauben verbunden. Jetzt kann die Prothese in situ verbracht und einerseits mit dem zuvor von proximal eingeführten Führungsdraht in der zentralen Öffnung des Schaftverankerungsteils und andererseits über das Prothesenschloss mit der Tibiaplateaukomponente verbunden werden. Der Führungsdraht wird etwa ein Zentimeter oberhalb der Trochanterspitze abgetrennt. Zusätzlich wird an dem Gelenkersatz- bzw. Schaftersatzteil lateralseitig noch eine L-Platte angebracht, über die das spätere Verschiebesegment bereits jetzt mit einer oder zwei Kleinfragmentschrauben fixiert wird.

Zur Verlängerung des verbliebenen Oberschenkelknochens wird mit einem geraden, auf den liegenden Führungsdraht abgestimmten kanülierten Fräser der Markraum zunächst bis zur geplanten Osteotomiehöhe stufenweise entsprechend dem Durchmesser des vorgesehenen Distraktionsmarknagels aufgefräst. Anschließend erfolgt die Knochendurchtrennung in minimal invasiver Technik, vorzugsweise mit einer Markraumsäge. Der Fräsvorgang wird nun bis zum Erreichen des Schaftverankerungsteils fortgesetzt. Eine eventuell vorliegende Krümmung des Knochens kann dabei überwunden werden, da nach der Osteotomie die beiden Knochensegmente in der Regel sehr kurz sind und sich zueinander ausrichten. Sobald das Schaftverankerungsteil erreicht ist, wird über den Führungsdraht ein kanüliertes Extraktionswerkzeug eingeführt, dessen Mehrkant am Ende in einen entsprechenden Innenmehrkant in dem Schaftverankerungsteil eingreift, so dass Rotationskräfte zur Lösung des Schaftverankerungsteils nach Entfernung der Formschlussverbindung zwischen dem Schaftverankerungsteil und dem Gelenkersatz- bzw. Schaftersatzteil übertragbar werden. Nun wird der Führungsdraht aus der Befestigungseinrichtung heraus geschraubt und eine Zugstange durch das kanülierte Werkzeug in das Gewinde der Befestigungsvorrichtung geschraubt. Damit kann nun auch eine Axialkraft auf den Schaftverankerungsteil ausgeübt und dieser nach proximal extrahiert werden. Eine Bohrschneide an der gelenkersatzteilabgewandten Seite des Schaftverankerungsteils kann hierbei kleinere intramedulläre Hindernisse abtragen. Anschließend wird der Distraktionsmarknagel durch den so gebildeten Knochenkanal in den Hohlraum des Schaftersatzteils eingeführt. Der Distraktionsmarknagel wird mit Bolzen oder Schrauben formschlüssig einerseits über die vorgesehenen Öffnungen mit dem Gelenkersatz- bzw. Schaftersatzteil und andererseits mit dem proximalen Femur verbunden. Bei Verwendung eines elektromotorischen Antriebs mit subkutaner Empfangsantenne kann es je nach Ausführungsform erforderlich sein, dass der Antrieb vorweg in das Schaftersatzteil eingeführt wird. Das Kabel wird hierzu mit Hilfe eines Fadens oder eines Drahtes vorweg durch den Kanal im proximalen Femur über die Osteotomie in die Öffnung des Gelenk- und Schaftersatzteils eingeführt und seitlich ausgeleitet.

Bei einem Ersatz des Hüftgelenks und des hüftgelenknahen Femurschaftanteils wird ähnlich vorgegangen. Sofern nicht eine Hemiarthroplastik vorgesehen ist, wird nach der En-Bloc-Resektion des erkrankten Knochenanteils des Femurs zunächst die Gelenkpfanne implantiert. Anschließend wird ein Führungsdraht interkondylär in der Frontalebene mittig und in der seitlichen Ebene in Verlängerung der distalen Femurschaftachse eingeführt und durch den Markraum bis in den Defekt vorgeschoben. Über diesen Führungsdraht wird der Markraum schrittweise so weit aufgefräst, wie es für das Einführen des Schaftverankerungsteils erforderlich ist. Das Gelenkersatzteil und das Schaftersatzteil werden je nach Defektgröße ausgewählt. In die gelenkersatzteilabgewandte Öffnung des Hohlraums des Schaftersatzteils wird nun das gerade, auf den Markraumdurchmesser abgestimmte, außen polierte Schaftverankerungsteil eingeführt und formschlüssig mit Bolzen oder Schrauben verbunden. Dann wird die Prothese in situ verbracht, wobei der Führungsdraht in die zentrale Öffnung des Schaftverankerungsteils eingeführt wird. Der Führungsdraht wird in der Notch abgetrennt. Zusätzlich wird an dem Gelenkersatzteil bzw. dem Schaftersatzteil lateral noch eine L-Platte angebracht, über die das spätere Verschiebesegment bereits jetzt mit einer oder zwei Kleinfragmentschrauben fixiert wird.

Nach Abschluss des Längenwachstums wird mit einem geraden, auf den liegenden Führungsdraht abgestimmten kanülierten Fräser der Markraum zunächst bis zur geplanten Osteotomiehöhe stufenweise entsprechend dem Durchmesser des vorgesehenen Distraktionsmarknagels aufgefräst. Anschließend erfolgt die Knochendurchtrennung in minimal invasiver Technik, vorzugsweise mit einer Markraumsäge. Der Fräsvorgang wird nun bis zum Erreichen des Schaftverankerungsteils der Prothese fortgesetzt. Eine eventuell vorliegende Krümmung des Knochens kann dabei überwunden werden, da nach der Osteotomie die beiden Knochensegmente in der Regel sehr kurz sind und sich zueinander ausrichten. Sobald das Schaftverankerungsteil erreicht ist, wird über den Führungsdraht ein kanüliertes Extraktionswerkzeug eingeführt, dessen Mehrkant am Ende in einen entsprechenden Innenmehrkant in dem Schaftverankerungsteil eingreift, so dass Rotationskräfte zur Lösung des Schaftverankerungsteils nach Entfernung der Formschlussverbindung zwischen dem Schaftverankerungsteil und dem Gelenkersatz- bzw. dem Schaftersatzteil übertragbar werden. Nun wird der Führungsdraht aus der Befestigungseinrichtung heraus geschraubt und eine Zugstange durch das kanülierte Werkzeug in das Gewinde der Befestigungsvorrichtung eingeschraubt. Damit kann nun auch eine Axialkraft auf das Schaftverankerungsteil ausgeübt und dieses nach distal extrahiert werden. Eine Bohrschneide an der gelenkersatzteilabgewandten Seite des Schaftverankerungsteils kann hierbei kleinere intramedulläre Hindernisse abtragen. Anschließend wird der Distraktionsmarknagel durch den so gebildeten Knochenkanal in den Hohlraum des Schaftersatzteils eingeführt. Der Distraktionsmarknagel wird mit Bolzen oder Schrauben formschlüssig einerseits über die vorgesehenen Öffnungen mit dem Gelenkersatz- bzw. Schaftersatzteil und andererseits mit dem distalen Femur verbunden. Bei Verwendung eines elektromotorischen Antriebs mit subkutaner Empfangsantenne kann es je nach Ausführungsform erforderlich sein, dass der Antrieb vorweg in das Schaftersatzteil eingeführt wird. Das Kabel wird hierzu mit Hilfe eines Fadens oder eines Drahtes vorweg durch den Kanal im distalen Femur über die Osteotomie in die Öffnung des Gelenk- und Schaftersatzteils eingeführt und seitlich ausgeleitet.

Anhand von Zeichnungen wird die Erfindung beispielsweise näher erläutert. Es zeigen:
- Fig. 1: in einem Längsschnitt schematisch eine implantierbare Prothese mit dem angrenzenden Knochenabschnitt,
- Fig. 2: in einer Teilansicht im Längsschnitt den Knochenabschnitt mit einer alternativen Ausgestaltung des gelenkersatzteilabgewandten Teils des Schaftverankerungsteils der Prothese,
- Fig. 3: in einer Teilansicht im Längsschnitt ein Extraktionswerkzeug im Eingriff mit dem Schaftverankerungsteil,
- Fig. 4: in einer Längsansicht schematisch den Ersatz des distalen Femurs mit einer ersten Ausführungsform eines anstelle des Schaftverankerungsteils eingesetzten Distraktionsmarknagels vor der Distraktion,
- Fig. 5: in einer Ansicht wie Fig. 3 den Zustand nach der Distraktion,
- Fig. 6: in einer Ansicht wie Fig. 3 den Ersatz des distalen Femurs mit einer zweiten Ausführungsform eines anstelle des Schaftverankerungsteils eingesetzten Distraktionsmarknagels vor der Distraktion,
- Fig. 7: in einer Ansicht wie Fig. 5 den Zustand nach der Distraktion,
- Fig. 8: in einer Ansicht wie Fig. 3 schematisch den Ersatz des proximalen Femurs mit der ersten Ausführungsform des Distraktionsmarknagels vor der Distraktion,
- Fig. 9: in einer Ansicht wie Fig. 7 den Zustand nach der Distraktion,
- Fig. 10: in einer Ansicht wie Fig. 7 schematisch den Ersatz des proximalen Femurs mit der zweiten Ausführungsform des Distraktionsmarknagels vor der Distraktion und
- Fig. 11: in einer Ansicht wie Fig. 9 den Zustand nach der Distraktion.

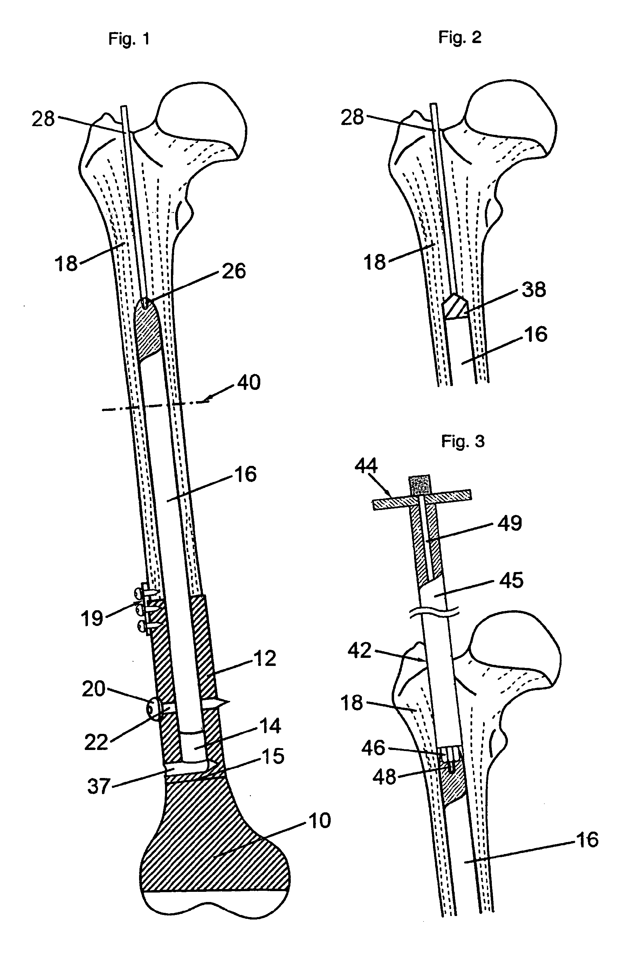

Die in Fig. 1 gezeigte implantierbare Prothese besteht aus einem Gelenkersatzteil 10, das mit einem Schaftersatzteil 12 für einen Austausch lösbar verbunden ist. Das Schaftersatzteil 12 ist über eine Platte 19 und Schrauben mit einem Knochenabschnitt 18 verbunden.

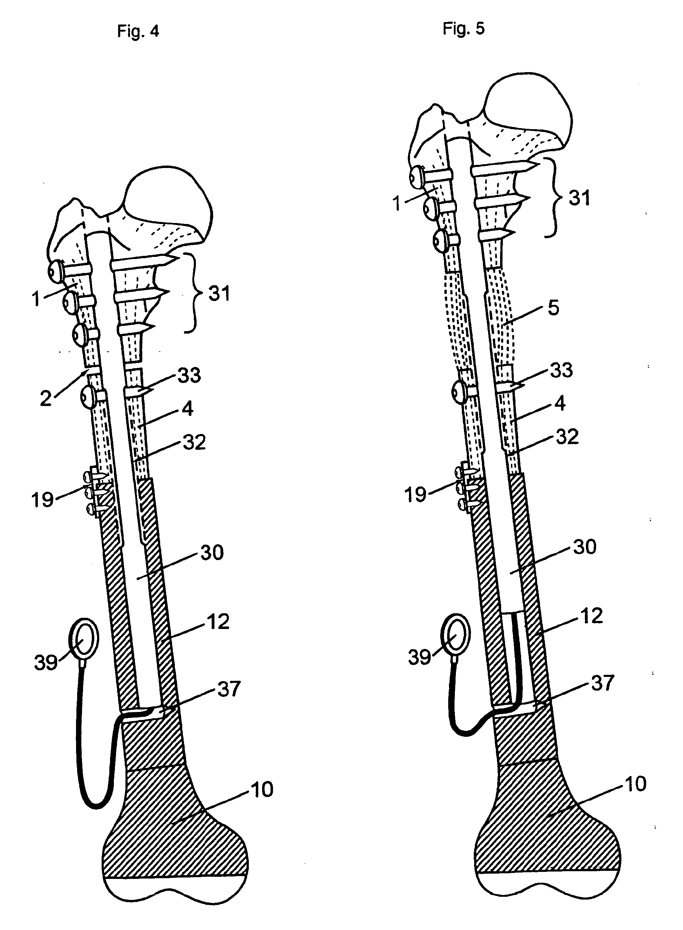

In dem Schaftersatzteil 12 ist ein Hohlraum 14 in Form einer zentralen Sackbohrung ausgebildet, die auf der Seite des Gelenkersatzteils 10 einen Boden 15 aufweist (in der Praxis wird man es sicher durchbohren, da man zweckmäßigerweise den Gelenkersatzteil und den Schaftersatzteil mit einer zentralen Schraube verbindet) und auf ihrer vom Gelenkersatzteil 10 abgewandten Seite offen ist. Die Innenwand des Hohlraums 14 ist mit einem gleitreibungsmindernden Belag versehen. In den Hohlraum 14 ist ein Schaftverankerungsteil 16 eingesetzt, dessen Wand poliert ist. Im Boden 15 des Schaftersatzteils 12 ist eine Seitenbohrung 37 für die Durchführung eines Anschlusselements vorgesehen, das mit seinem einen Ende mit einem Antrieb eines anstelle des Schaftverankerungsteils 16 eingesetzten Distraktionsmarknagels 30 (Fig. 4 bis 11) und mit seinem auf der Prothesenaußenseite befindlichen Ende mit einem Subkutanempfänger zur Energieeinspeisung in den Antrieb verbindbar ist. Das Schaftverankerungsteil 16 ist mit dem Schaftersatzteil 12 mittels eines Schraubenbolzens 20 verbunden, der sich durch eine Querbohrung 22 im Schaftersatzteil 12 und im Schaftverankerungsteil 16 erstreckt.

Das Schaftverankerungsteil 16 hat an seinem vom Gelenkersatzteil 10 abgewandten Ende eine Öffnung als Befestigungseinrichtung 26, mit der eine Ortungseinrichtung 28 in Form eines Führungsdrahts lösbar in Eingriff steht, beispielsweise durch eine Gewindeverbindung. Der Führungsdraht 28 erstreckt sich von dem Schaftverankerungsteil 16 durch den Knochenabschnitt 18 hindurch und aus ihm heraus.

Bei der in Fig. 1 gezeigten Ausführungsform bildet der Führungsdraht 28 die Führung für einen außerhalb des Knochenabschnitts 18 befindlichen Fräser zum Fräsen eines Kanals 42 (Fig. 3) bis zum freien Ende des Schaftverankerungsteils 16 hin, das dann nach Lösen des Bolzens 20 durch den gefrästen Kanal 42 entfernt und durch einen Marknagel 30 (Fig. 4 bis 11) ersetzt werden kann, der durch Querbolzen 31 bzw. 3 (Fig. 4 bis 11) entsprechend fixiert wird, nachdem längs einer strichpunktiert gezeigten Trennebene 40 eine Osteotomie des Knochens mit Hilfe einer nicht gezeigten Innensäge vorgenommen worden ist.

Bei der in Fig. 2 gezeigten Ausführung steht von dem Schaftverankerungsteil 16 ein Schneidbohrer 38 vor, der bei Drehung des Schaftverankerungsteils 16 unter Zug eventuell vorhandene Vorsprünge an der Wand des gefrästen Kanals 42 (Fig. 3) wegnimmt und so das Herausziehen des Schaftverankerungsteils 16 aus dem Knochenabschnitt 18 und das anschließende Einbringen eines Distraktionsmarknagels 30 (Fig. 4 bis 11) erleichtert.

Zum Herausziehen des Schaftverankerungsteils 16 aus dem Knochenabschnitt 18 an dessen gelenkersatzteilabgewandter Seite hat dieses, wie in Fig. 3 gezeigt ist, stirnseitig einen Innenmehrkant 46 und eine daran anschließende Innengewindebohrung 48 für den Eingriff mit einem durch den Kanal 42 passenden hülsenförmigen Verbindungselement 45 eines außerhalb des Knochenabschnitts 18 befindlichen Werkzeugs 44. Das Verbindungselement 45 greift mit einem endseitigen Außenmehrkant in den Innenmehrkant 46 des Schaftverankerungsteils 16 formschlüssig ein. Eine durch das Verbindungselement 45 drehungsfrei hindurchgehende Zugstange 49 ist mit ihrem endseitigen Außengewinde in die Innengewindebohrung 48 des Schaftersatzteils 16 eingeschraubt. Dadurch kann das Werkzeug 44, wenn es gedreht wird, eine Zugkraft und ein Drehmoment für ein leichtes Extrahieren des Schaftverankerungsteils 16 übertragen.

In Fig. 4 bis 11 werden Varianten der Knochenverlängerung unter Verwendung eines bekannten anstelle des extrahierten Schaftverankerungsteils 16 (Fig. 1 bis 3) eingesetzten Distraktionsmarknagels 30 beschrieben.

Fig. 4 und 5 zeigen den Ersatz des distalen Femurknochens zum Beispiel nach Resektion eines Knochentumors. Dargestellt sind ein Gelenkersatzteil 10 und ein Schaftersatzteil 12. Von proximal ist ein Distraktionsmarknagel 30 mit einem Langloch 32 formschlüssig aber axial verschieblich in das Schaftersatzteil 12 eingeführt. Der Distraktionsmarknagel 30 ist zum Beispiel mit einer oder, vorteilhafter, wie dargestellt mit drei Schrauben 31 in dem proximalen Femurknochen 1 (in Fig. 1 bis 3 Knochenabschnitt 18) fixiert, wodurch auch sehr proximale Osteotomien 2 (in Fig. 1 bis 3 Trennebene 40) möglich werden. Die Fixierung distal der Osteotomie 2 erfolgt im proximalen Ende des Langlochs 32 ebenfalls mit einer Schraube 33. Über diese Schraube 33 kann mit einem Antrieb im Marknagel 30, z. B. mit einem Getriebemotor, eine axiale Distraktionskraft auf den Knochen ausgeübt und der Knochen so nach der Kallusdistraktionsmethode im Osteotomiespalt 2 durch Bildung von Kallus 5 verlängert werden. Die physiologische Antekurvation des Femurs stellt keinen Hindernisgrund für die Einbringung eines geraden Marknagels 30 dar, da der Femur an der Osteotomie 2 begradigt wird. Durch die Seitenbohrung 37 wird ein Elektrokabel zur Verbindung eines innenliegenden Antriebs mit einer im Unterhautfettgewebe liegenden Antenne 39 ausgeleitet, über die dann von außen Energie eingekoppelt werden kann.

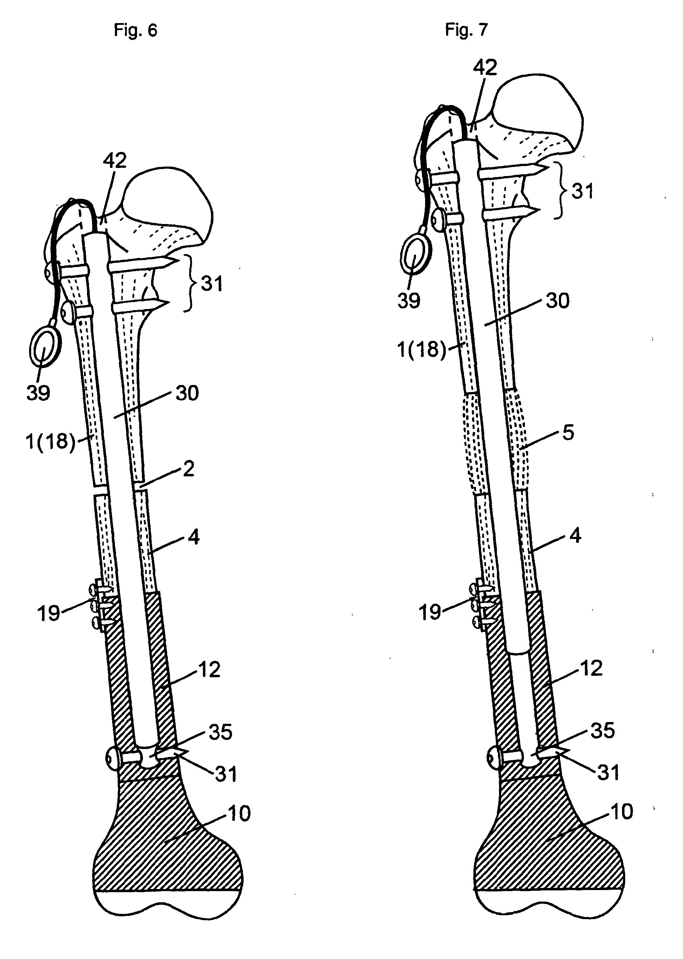

Fig. 6 und 7 entsprechen im Wesentlichen Fig. 4 und 5. Die Verlängerung erfolgt hier mit einem Distraktionsmarknagel 30, der einen Teleskopmechanismus 35 aufweist und der einerseits mit Schrauben 31 im proximalen Femurknochen 1 (in Fig. 1 bis 3 Knochenabschnitt 18) verankert ist und andererseits formschlüssig mit einer weiteren Schraube 31 in dem Schaftersatzteil 12 fixiert ist. Zur Erhöhung der Festigkeit in Schaftmitte, also der Stelle der maximalen Biegebeanspruchung, kann der Teleskopmarknagel 30 so weit in das Schaftersatzteil 12 versenkt werden, dass er auch im ausgefahrenen Zustand noch ausreichend weit mit dem großen Durchmesser in dem Schaftersatzteil 12 verbleibt. Das Kabel für die Antenne 39 ist bei dieser Ausgestaltung durch den ausgefrästen Kanal 42 abgeleitet.

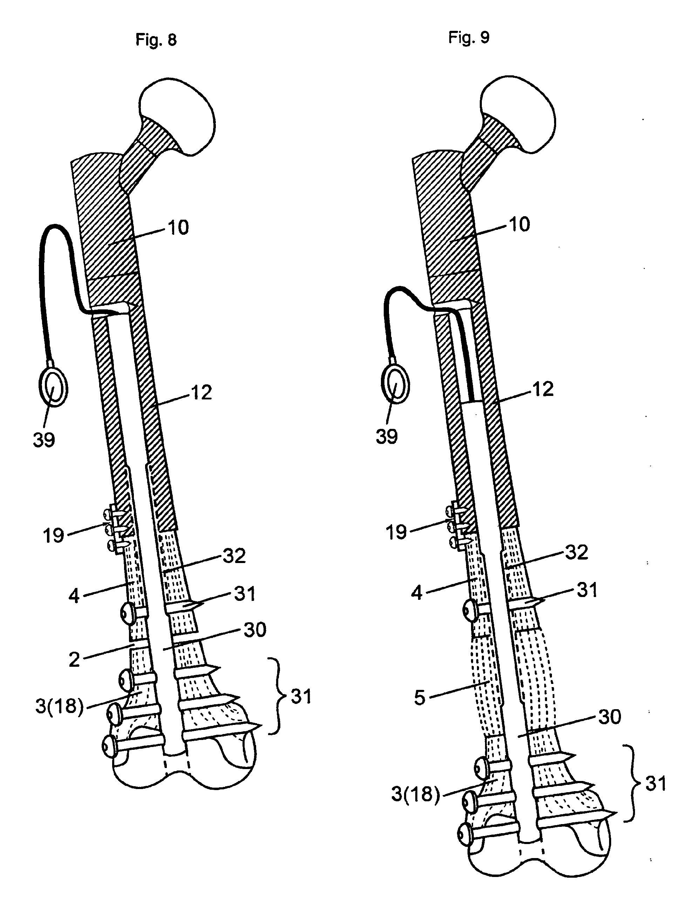

Fig. 8 und 9 zeigen den Ersatz des proximalen Femurknochens zum Beispiel nach Resektion eines Knochentumors. Hier wird von distal vom Kniegelenk aus durch den distalen Femurknochen 3, ein Distraktionsmarknagel 30 mit einem Langloch 32 in das Schaftersatzteil 12 axial verschieblich eingeführt und einerseits mit mehreren Schrauben 31 im distalen Femur 3 (entspricht funktionsgemäß dem Knochenabschnitt 18 von Fig. 1 bis 3) und andererseits mit einer weiteren Schraube 31 im distalen Langlochende und dem verbliebenen Femurschaft 4 verankert, der über Verbindungseinrichtungen 19 in Form einer L-förmigen Platte mit dem Schaftersatzteil 12 verbunden ist. Über das Langloch 32 im Marknagel kann mit einem Antrieb, beispielsweise mit einem Getriebemotor, innerhalb des Marknagels 30 eine axiale Distraktionskraft auf den Knochen ausgeübt und der Knochen so im Osteotomiespalt 2 (Trennebene 40 von Fig. 1 bis 3) nach der Kallusdistraktionsmethode (Kallus 5) verlängert werden.

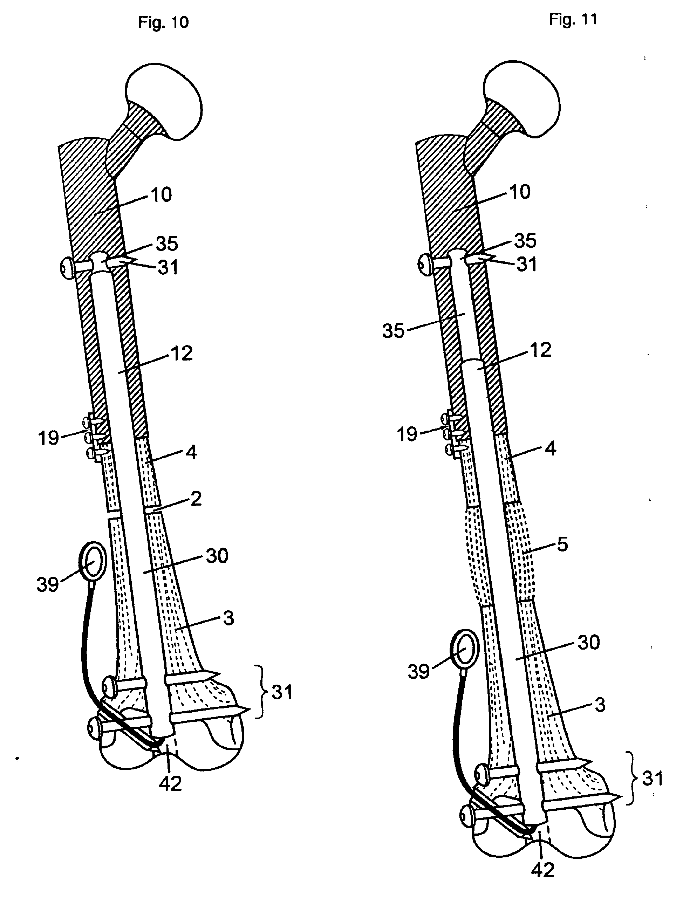

Fig. 10 und 11 entsprechen im Wesentlichen Fig. 8 und 9. Hier erfolgt die Verlängerung (Kallus 5) jedoch mit einem Distraktionsmarknagel 30 mit einem Teleskopmechanismus 35 analog zu Fig. 6 und 7, der mit Schrauben 31 im distalen Femur 3 und mit einer weiteren Schraube 31 in dem Schaftersatzteil 12 fixiert ist. Zur Erhöhung der Festigkeit in Schaftmitte, also der Stelle der maximalen Biegebeanspruchung, kann der Teleskopmarknagel 30 auch hier so weit in das Schaftersatzteil versenkt werden, dass er auch im ausgefahrenen Zustand noch ausreichend weit mit dem großen Durchmesser in dem Schaftersatzteil 12 verbleibt. Das Kabel für die Antenne 39 ist bei dieser Ausgestaltung durch den ausgefrästen Kanal 42 abgeleitet.

## Patentansprüche

1. Implantierbare Prothese zum Ersatz des menschlichen Hüft- oder Kniegelenks und der angrenzenden Knochenabschnitte
- mit einem Gelenkersatzteil (10),
- mit einem Schaftersatzteil (12), das mit dem Gelenkersatzteil (10) verbindbar ist oder ein Stück damit bildet und einen zentralen Hohlraum (14) hat, und
- mit einem stabförmigen Schaftverankerungsteil (16), das in den Hohlraum (14) im-Schaftersatzteil (12) einsetzbar ist,
**dadurch gekennzeichnet, dass** das Schaftverankerungsteil (16) auf der gelenkersatzteilabgewandten Seite
- eine Ortungseinrichtung (28) zum Ausbilden eines Kanals (42) durch den Knochenabschnitt (18) auf der gelenkersatzteilabgewandten Seite von außen her axial fluchtend zu dem Schaftverankerungsteil (16) und bis zu diesem hin sowie
- eine Befestigungseinrichtung (26) für den Eingriff mit einem außerhalb des Knochenabschnitts (18) befindlichen Werkzeug (44) durch den Kanal (42) hindurch zum Ausüben wenigstens einer Zugkraft auf das Schaftverankerungsteil (16) aufweist, um es aus dem Knochenabschnitt (18) durch den Kanal (42) hindurch entfernen zu können.

2. Implantierbare Prothese nach Anspruch 1, **dadurch gekennzeichnet, dass** die Ortungseinrichtung (28) eine Strahlungsquelle ist.

3. Implantierbare Prothese nach Anspruch 1, **dadurch gekennzeichnet, dass** die Ortungseinrichtung (28) ein aus der Befestigungseinrichtung (26) zentral vorstehendes drahtförmiges Führungselement ist, das durch den Knochenabschnitt (18) hindurchgeht und aus diesem nach außen herausragt.

4. Implantierbare Prothese nach Anspruch 3, **dadurch gekennzeichnet, dass** das Führungselement (28) mit der Befestigungseinrichtung (26) lösbar verbindbar ist.

5. Implantierbare Prothese nach einem der vorhergehenden Ansprüche, **dadurch gekennzeichnet, dass** das Schaftverankerungsteil (16) so ausgebildet ist, dass auf es durch das Werkzeug (44) auch ein Drehmoment übertragbar ist.

6. Implantierbare Prothese nach einem der vorhergehenden Ansprüche, **dadurch gekennzeichnet, dass** die Befestigungseinrichtung (26) eine Innengewindebohrung (48) aufweist.

7. Implantierbare Prothese nach Anspruch 6, **dadurch gekennzeichnet, dass** im Schaftverankerungsteil (16) werkzeugseitig vor der Innengewindebohrung (48) ein Innenmehrkant (46) vorgesehen ist.

8. Implantierbare Prothese nach einem der vorhergehenden Ansprüche, **dadurch gekennzeichnet, dass** das Schaftverankerungsteil (16) auf der Werkzeugbefestigungsseite eine Bohrschneide (38) aufweist.

9. Implantierbare Prothese nach einem der vorhergehenden Ansprüche, **dadurch gekennzeichnet, dass** die Oberfläche des Schaftverankerungsteils (16) eine Oberflächenrautiefe von 0,1 µm oder weniger aufweist.

10. Implantierbare Prothese nach einem der vorhergehenden Ansprüche, **dadurch gekennzeichnet, dass** die Innenwand des Hohlraums (14) im Schaftersatzteil (12) gleitreibungsmindernd ausgestaltet ist.

11. Implantierbare Prothese nach einem der vorhergehenden Ansprüche, **dadurch gekennzeichnet, dass** das Schaftersatzteil (12) an seinem gelenkersatzteilseitigen Ende eine in den Hohlraum (14) mündende Seitenbohrung (37) aufweist.

12. Implantierbare Prothese nach einem der vorhergehenden Ansprüche, **dadurch gekennzeichnet, dass** an dem Schaftersatzteil (12) im Bereich seines gelenkersatzteilabgewandten Endes Verbindungseinrichtungen (19) zur Fixierung an einem angrenzenden Knochenabschnitt anbringbar sind.

13. Implantierbare Prothese nach einem der vorhergehenden Ansprüche, **dadurch gekennzeichnet, dass** das Schaftverankerungsteil (16) über seiner Länge einen gleichbleibenden und/oder einen zum Gelenkersatzteil (10) hin verjüngten Querschnitt hat.

14. Implantierbare Prothese nach einem der vorhergehenden Ansprüche, **dadurch gekennzeichnet, dass** das Schaftverankerungsteil (16) ein Stabilisator oder ein Marknagel ist.

## Claims

1. An implantable prosthesis for replacing a human hip or knee joint and the adjoining bone sections, comprising
- a joint replacement part (10);
- a shaft replacement part (12) being connectable to the joint replacement part (10) or integral therewith and having a central cavity (14); and
- a rod-shaped shaft anchoring part (16) being insertable into the cavity (14) in the shaft replacement part (12);
**characterized in that** the shaft anchoring part (16) at the side facing away from the joint replacement part comprises
- locating means (28) for forming a channel (42) through the bone section (18) at the side facing away from the joint replacement part from the outside, the channel (42) being axially aligned with the shaft anchoring part (16) and reaching up to the shaft anchoring part (16); and
- attachment means (26) for engagement with a tool (44) located outside the bone section (18) through the channel (42) for exerting at least a traction force on the shaft anchoring part (16) for removing the shaft anchoring part (16) from the bone section (18) through the channel (42).

2. The implantable prosthesis according to claim 1, **characterized in that** the locating means (28) is a radiation source.

3. The implantable prosthesis according to claim 1, **characterized in that** the locating means (28) is a wire-shaped guide element protruding centrally from the attachment means (26), wherein the wire-shaped guide element passes through the bone section (18) and protrudes therefrom to the outside.

4. The implantable prosthesis according to claim 3, **characterized in that** the guide element (28) can be detachably connected to the attachment means (26).

5. The implantable prosthesis according to one of the preceding claims, **characterized in that** the shaft anchoring part (16) is formed such that also a torque can be transmitted by the tool (44) to the shaft anchoring part (16).

6. The implantable prosthesis according to one of the preceding claims, **characterized in that** the attachment means (26) has an internally threaded bore (48).

7. The implantable prosthesis according to claim 6, **characterized in that** a polygonal socket (46) is provided in the shaft anchoring part (16) in front of the internally threaded bore (48) at the tool side.

8. The implantable prosthesis according to one of the preceding claims, **characterized in that** the shaft anchoring part (16) has a drill cutter (38) at the tool attachment side.

9. The implantable prosthesis according to one of the preceding claims, **characterized in that** the surface of the shaft anchoring part (16) has a surface roughness depth of 0.1 µm or less.

10. The implantable prosthesis according to one of the preceding claims, **characterized in that** the inner wall of the cavity (14) in the shaft replacement part (12) is designed to be slide friction reducing.

11. The implantable prosthesis according to one of the preceding claims, **characterized in that** the shaft replacement part (12), at its end facing the joint replacement part, has a lateral bore (37) opening into the cavity (14).

12. The implantable prosthesis according to one of the preceding claims, **characterized in that** connection means (19) can be mounted at the shaft replacement part (12) in the region of its end facing away from the joint replacement part for fixing it to an adjoining bone section.

13. The implantable prosthesis according to one of the preceding claims, **characterized in that** the shaft anchoring part (16) over its length has an invariable diameter and/or a diameter tapered towards the joint replacement part (10).

14. The implantable prosthesis according to one of the preceding claims, **characterized in that** the shaft anchoring part (16) is a stabilizer or a medullary nail.

## Revendications

1. Prothèse implantable pour le remplacement de l'articulation d'une hanche ou d'un genou chez l'homme et des sections d'os limitrophes
- comportant une partie de remplacement articulée (10),
- comportant une partie de remplacement de tige (12), qui peut être reliée à la partie de remplacement articulée (10) ou qui forme avec elle une pièce et a une cavité centrale (14),
- comportant une partie d'ancrage de tige en forme de barre (16), qui peut être introduite dans la cavité (14) dans la partie de remplacement de tige (12),
**caractérisée en ce que** la partie d'ancrage de tige (16) sur le côté opposé à la partie de remplacement articulée présente
- un dispositif de localisation (28) pour la constitution d'un canal (42) à travers la section d'os (18) sur le côté opposé à la partie de remplacement articulée depuis l'extérieur en alignement axial avec la partie d'ancrage de tige (16) et en direction de celle-ci ainsi
- qu'un dispositif de fixation (26) pour l'intervention avec un outil (44) qui se trouve à l'extérieur de la section d'os (18) à travers le canal (42) pour exercer au moins une force de traction sur la partie d'ancrage de tige (16), afin de pouvoir l'éloigner de la section d'os (18) à travers le canal (42).

2. Prothèse implantable selon la revendication 1, **caractérisée en ce que** le dispositif de localisation (28) est une source de rayonnement.

3. Prothèse implantable selon la revendication 1, **caractérisée en ce que** le dispositif de localisation (28) est un élément de guidage, en forme de fil saillant au centre du dispositif de fixation (26), qui traverse la section d'os (18) et dépasse vers l'extérieur depuis celle-ci.

4. Prothèse implantable selon la revendication 3, **caractérisée en ce que** l'élément de guidage (28) peut être relié de manière amovible au dispositif de fixation (26).

5. Prothèse implantable selon l'une des revendications précédentes, **caractérisée en ce que** la partie d'ancrage de tige (16) est configurée de sorte qu'un couple de rotation peut également lui être transmis par le biais de l'outil (44).

6. Prothèse implantable selon l'une des revendications précédentes, **caractérisée en ce que** le dispositif de fixation (26) présente un taraudage interne (48).

7. Prothèse implantable selon la revendication 6, **caractérisée en ce que** dans la partie d'ancrage de tige (16) est prévu côté outil en amont du taraudage interne (48) un polygone interne (46).

8. Prothèse implantable selon l'une des revendications précédentes, **caractérisée en ce que** la partie d'ancrage de tige (16) présente un taillant (38) sur le côté de fixation de l'outil.

9. Prothèse implantable selon l'une des revendications précédentes, **caractérisée en ce que** la surface de la partie d'ancrage de tige (16) présente une profondeur de rugosité de surface de 0,1 µm ou moins.

10. Prothèse implantable selon l'une des revendications précédentes, **caractérisée en ce que** la paroi interne de la cavité (14) est réalisée dans la partie de remplacement de tige (12) de manière à réduire le frottement du glissement.

11. Prothèse implantable selon l'une des revendications précédentes, **caractérisée en ce que** la partie de remplacement de tige (12) présente au niveau de son extrémité côté partie de remplacement articulée un alésage latéral (37) débouchant dans la cavité (14).

12. Prothèse implantable selon l'une des revendications précédentes, **caractérisée en ce que** des dispositifs de liaison (19) pour la fixation à une section d'os limitrophe peuvent être installés au niveau de la partie de remplacement de tige (12) dans la zone de son extrémité opposée à la partie de remplacement articulée.

13. Prothèse implantable selon l'une des revendications précédentes, **caractérisée en ce que** la partie d'ancrage de tige (16) a sur sa longueur une section transversale constante et/ou qui se rétrécit en direction de la partie de remplacement articulée (10).

14. Prothèse implantable selon l'une des revendications précédentes, **caractérisée en ce que** la partie d'ancrage de tige (16) est un stabilisateur ou un clou centro-médullaire.
